# EUROPEAN PATENT SPECIFICATION

(11) **EP 0 372 503 B1**
(45) Date of publication and mention of the grant of the patent: **10.03.1999**
(21) Application number: 89122451.1
(22) Date of filing: 06.12.1989
(51) Int. Cl.: C07K 7/06, A61K 38/55

(54) **Anticoagulant peptides**
Antikoagulierende Peptide
Peptides anticoagulants

(30) Priority: 07.12.1988 US 281121
(43) Date of publication of application: 13.06.1990
(73) Proprietor: MERRELL PHARMACEUTICALS INC., Cincinnati, Ohio 45215-6300 (US)
(72) Inventor: Krstenansky, John L., Cincinnati Ohio 45208 (US)
(74) Representative: VOSSIUS & PARTNER

(56) References cited:
- EP-A- 0 276 014
- THROMBOSIS RESEARCH, vol. 52, 1988, pages 137-141, Pergamon Press plc,; J.L. KRSTENNANSKY et al.: "Comparison of hirudin and hirudin PA C-terminal fragments and related analogs as antithrombin agents"
- J. MED. CHEM., vol. 30, 1987, 1688-1691, American Chemical Society; J.L. KRSTENANSKY et al.: "Anticoagulant peptides: Nature of the interaction of the C-terminal region of hirudin with a noncatalytic binding site on thrombin"

## Description

This invention relates to novel peptides which are useful anticoagulant agents.

Anticoagulants are useful therapeutic agents in the pharmacological treatment of, for example, acute deep venous thrombosis, pulmonary embolism, acute arterial embolization of the extremities, myocardial infarction, and disseminated intravascular coagulation. Prophylactic administration of anticoagulants is believed to prevent a recurrence of embolism in patients with rheumatic or arteriosclerotic heart disease and to prevent certain thromboembolic complications of surgery. Administration of anticoagulants has also been indicated in the treatment of coronary artery and cerebrovascular disease. Arterial thrombosis, particularly in arteries supplying the heart muscle and brain, is a leading cause of death.

Hirudin is a 65 residue polypeptide isolated from the salivary glands of leeches. It is an anticoagulant agent, which is a thrombin specific inhibitor. Although quite potent, clinical use of hirudin isolated from leech extracts seems unlikely because of its limited quantity, expense and allergic reactions which commonly follow administration of any foreign protein of this size.

Thrombosis Research (1988), vol. 52, pages 137-141 discloses Hirudin and Hirudin PA C-terminal fragments and related analogues and presents an assesment of their potency as antithrombin agents.

J. Med. Chem. (1987), vol. 30, pages 1688-1691 discloses a series of 20 C-terminal fragment analogues of Hirudin and reports on their ability to inhibit thrombin-induced dot formation.

Applicants have discovered a specific region of hirudin that is responsible, at least in part, for its anticoagulant activity. This region has been chemically synthesized and certain of its analogs appear to bind to the recognition site of thrombin but not the enzymatic cleavage site which is spatially separate. Binding of the synthetic peptides competitively prevents binding of the fibrinogen to the recognition site of thrombin, a prerequisite to fibrin production and clot formation. The peptides of this invention possess significant anticoagulant activity and their unusual ability to bind only to the recognition site without binding to the cleavage site of thrombin may allow for a scientifically interesting and therapeutically significant adjunct to anticoagulant therapy.

Peptide derivatives of the formula

X-A₁-A₂-A₃-A₄-A₅-A₆-A₇-A₈-A₉-A₁₀-Y

wherein
X is a hydrogen, one or two alkyl groups selected from the group methyl, ethyl, propyl, isopropyl, butyl, isobutyl, and tert-butyl, or one or two acyl groups selected from the group acetyl, and succinyl;
A₁ is a bond;
A₂ is Tyr;
A₃ is Glu;
A₄ is Pro, Pip, Azd, or Tiq;
A₅ is Ile;
A₆ is Pro, Pip, Azd, or Tiq;
A₇ is Glu;
A₈ is Glu;
A₉ is a dipeptide Ala-Cha;
A₁₀ is D-Glu; and
Y is a carboxy terminal residue selected from OH, (C₁-C₆)alkoxy, and amino, with the proviso that A₄ and A₆ cannot both be a Pro.

The following common abbreviations of the amino acids are used throughout this specification:
Gly - glycine
Ala - alanine
Val - valine
Leu - leucine
Ile - isoleucine
Cha - cyclohexylalanine
Orn - ornithine
Pro - proline
Phe - phenylalanine
Trp - tryptophan
Met - methionine
Ser - serine
Thr - threonine
Cys - cysteine
Tyr - tyrosine
Asn - asparagine
Gln - glutamine
Asp - aspartic acid
Glu - glutamic acid
Lys - lysine
Arg - arginine
His - histidine
Nle - norleucine
Hyp - hydroxyproline
Glt - glutaryl
Mal - maleyl
Npa -β-(2-naphthyl)alanine
3,4-dehydroPro - 3,4-dehydroproline
Tyr(SO₃H) - tyrosine sulfate
Pgl - phenylglycine
NMePgl - N-methyl-phenylglycine
Sar - sarcosine (N-methylglycine)
pSubPhe - para substituted phenylalanine
SubPhe - ortho, meta, or para, mono- or di- substituted phenylalanine
DAla - D-alanine
Ac - acetyl
Suc - succinyl
pClPhe - para-chloro-phenylalanine
pNO₂Phe - para-nitro-phenylalanine
Pip - L-2-pipecolic acid
Azd - L-azetidine-2-carboxylate
Tiq - L-1,2,3,4-tetrahydroisoquinoline-3-carboxylate
Thz - L-thiazolidine-4-carboxylate

An alkyl group and the alkyl portion of an alkoxy group is taken to include straight, branched, or cyclic alkyl groups, for example, methyl, ethyl, propyl, isopro- pyl, butyl, isobutyl, tert-butyl, pentyl, isopentyl, sec-pentyl, cyclopentyl, hexyl, isohexyl, cyclohexyl and cyclopentylmethyl. An acyl group of from 2 to 10 carbon atoms is taken to include straight, branched, cyclic, saturated and unsaturated acyl groups having 1 or 2 carbonyl moieties per group, for example, acetyl, benzoyl succinyl, maleyl, and glutaryl . A halogen group is a fluoro, chloro, bromo or iodo group.

The term "any amino acid" as used herein includes the naturally occurring amino acids as well as other "non-protein" α-amino acids commonly utilized by those in the peptide chemistry arts when preparing synthetic analogs of naturally occurring peptides. The naturally occurring amino acids are glycine, alanine, valine, leucine, isoleucine, serine, methionine, threonine, phenylalanine, tyrosine, tryptophan, cysteine, proline, histidine, aspartic acid, asparagine, glutamic acid, glutamine, arginine, ornithine, and lysine. Examples of "non-protein" α-amino acids are norleucine, norvaline, alloisoleucine, homoarginine, thiaproline, dehydroproline, hydroxyproline (Hyp), homoserine, cyclohexylglycine (Chg), α-amino-n-butyric acid (Aba), cyclohexylalanine (Cha), aminophenylbutyric acid (Pba), phenylalanines substituted at the ortho, meta, or paraposition of the phenyl moiety with one or two of the following, a (C₁-C₄) alkyl, (C₁-C₄) alkoxy, halogen, or nitro groups or substituted with a methylenedioxy group, β-2- and 3-thienylalanine, β-2- and 3-furanylalanine, β-2-, 3-, and 4-pyridylalanine, β-(benzothienyl-2- and 3-yl)alanine, β-(1- and 2-naphthyl)alanine, O-alkylated derivates of serine, threonine, or tyrosine, S-alkylated cysteine, the O-sulfate ester of tyrosine, 3,5-diiodotyrosine and the D-isomers of the naturally occurring amino acids.

The term "lipophilic amino acid" includes Tyr, Phe, Leu, Nle, Ile, Val, His and Pro.

The natural amino acids with the exception of glycine, contain a chiral carbon atom. Unless otherwise specifically indicated, the optically active amino acids, referred to herein, are of the L-configuration. For example, any of the amino acids of the A₁ to A₁₀ group can be of the D- or L-configuration. As is customary, the structure of peptides written out herein is such that the amino terminal end is on the left side of the chain and the carboxy terminal end is on the right side of the chain.

The polypeptides of formula 1 can form pharmaceutically acceptable salts with any non-toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid. Salts of the carboxy terminal amino acid moiety include the non-toxic carboxylic acid salts formed with any suitable inorganic or organic bases. Illustratively, these salts include those of alkali metals, as for example, sodium and potassium; alkaline earth metals, such as calcium and magnesium; light metals of Group IIIA including aluminum; and organic primary, secondary and tertiary amines, as for example, trialkylamines, including triethylamine, procaine, dibenzylamine, 1-ethenamine, N,N'-dibenzylethylenediamine, dihydroabietylamine, N-(lower)alkylpiperidine, and any other suitable amine.

As with any generic group of chemical compounds, certain groups are preferred. Applicants prefer those peptide derivatives of formula 1 wherein
X is succinyl.
Also preferred are those formula 1 compounds wherein
Y is OH.

The proteins of this invention can be prepared by a variety of procedures readily known to those skilled in the art. Such procedures include the solid phase sequential and block synthesis, gene cloning and combinations of these techniques. The solid phase sequential procedure can be performed using established automated methods such as by use of an automated peptide synthesizer. In this procedure an α-amino protected amino acid is bound to a resin support. The resin support employed can be any suitable resin conventionally employed in the art for the solid phase preparation of polypeptides, preferably polystyrene which has been cross-linked with from 0.5 to about 3 percent divinyl benzene, which has been either chloromethylated or hydroxymethylated to provide sites for ester formation with the initially introduced α-amino protected amino acid.

An example of a hydroxymethyl resin is described by Bodanszky, et al., Chem. Ind. (London) 38, 1597-98 (1966). A chloromethylated resin is commercially available from Bio Rad Laboratories, Richmond, California, and the preparation of such a resin is described by Stewart et al., "Solid Phase Peptide Synthesis" (Freeman & Co., San Francisco 1969), Chapter 1, pp. 1-6. The protected amino acid can be bound to the resin by the procedure of Gisin, Helv. Chim.Acta, 56, 1476 (1973). Many resin bound, protected amino acids are commercially available. As an example, to prepare a polypeptide of this invention wherein the carboxy terminal end is a Thr residue, a tert-butyloxycarbonyl (Boc) protected Thr bound to a benzylated, hydroxymethylated phenylacetamidomethyl (PAM) resin can be used and is commercially available.

Following the coupling of the α-amino protected amino acid to the resin support, the protecting group is removed using any suitable procedure such as by using trifluoroacetic acid in methylene chloride, trifluoroacetic acid alone, or HCl in dioxane. The deprotection is carried out at a temperature of between 0°C and room temperature. Other standard cleaving reagents and conditions for removal of specific α-amino protecting groups may be used. After removal of the α-amino protecting group the other amino protected amino acids are coupled step-wise in the desired order. Alternatively, multiple amino acid groups may be coupled by the solution method prior to coupling with the resin supported amino acid sequence.

The α-amino protecting group employed with each amino acid introduced into the polypeptide sequence may be any such protecting group known to the art. Among the classes of α-amino protecting groups contemplated are (1) acyl type protecting groups such as: formyl, trifluoroacetyl, phthalyl, toluenesulfonyl (tosyl), benzenesulfonyl, nitro-phenylsulfenyl, tritylsulfenyl, o-nitrophenoxyacetyl and α-chlorobutyryl; (2) aromatic urethane type protecting groups such as benzyloxycarbonyl and substituted benzyloxycarbonyl, such as p-chlorobenzyloxycarbonyl, p-nitrobenzyloxycarbonyl, p-bromobenzyloxycarbonyl, p-methoxybenzyloxycarbonyl, 1-(p-biphenylyl)-1-methylethoxycarbonyl, α,α-dimethyl-3,5-dimethoxybenzyloxycarbonyl and benzhydryloxycarbonyl; (3) aliphatic urethane protecting groups such as tert-butyloxycarbonyl (Boc), diisopropylmethoxycarbonyl, isopropyloxycarbonyl, ethoxycarbonyl and allyloxycarbonyl; (4) cycloalkyl urethane type protecting groups such as cyclopentyloxycarbonyl, adamantyloxycarbonyl and cyclohexyloxycarbonyl; (5) thio urethane type protecting groups such as phenylthiocarbonyl; (6) alkyl type protecting groups such as triphenylmethyl (trityl) and benzyl; and (7) trialkylsilane groups such as trimethylsilane. The preferred α-amino protecting group is tert-butyloxycarbonyl.

The selection of an appropriate coupling reagent is within the skill of the art. A particularly suitable coupling reagent where the amino acid to be added is Gln, Asn or Arg is N,N'-diisopropylcarbodiimide and l-hydroxy-benzotriazole. The use of these reagents prevents nitrile and lactam formation. Other coupling agents are (1) carbodiimides (e.g., N,N'-dicyclohexylcarbodiimide and N-ethyl-N'-(γ-dimethylaminopropylcarbodiimide)); (2) cyanamides (e.g., N,N-dibenzylcyanamide); (3) ketenimines; (4) isoxazolium salts (e.g., N-ethyl-5-phenyl-isoxazolium-3'-sulfonate); (5) monocyclic nitrogen containing heterocyclic amides of aromatic character containing one through four nitrogens in the ring such as imidazolides, pyrazolides, and 1,2,4-triazolides. Specific heterocyclic amides that are useful include N,N'-carbonyldiimidazole and N,N-carbonyl-di-1,2,4-triazole; (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., Boc-Ala-O-Ala-Boc) and (8) nitrogen containing heterocyclic compounds having a hydroxy group on one ring nitrogen (e.g., N-hydroxyphthalimide, N-hydroxysuccinimide and 1-hydroxybenzotriazole). Other activating reagents and their use in peptide coupling are described by Kapoor, J. Pharm. Sci., 59, pp. 1-27 (1970). Applicants prefer the use of the symmetrical anhydride as a coupling reagent for all amino acids except Arg, Asn and Gln.

Each protected amino acid or amino acid sequence is introduced into the solid phase reactor in about a four-fold excess and the coupling is carried out in a medium of dimethylformamide: methylene chloride (1:1) or in dimethylformamide alone or preferably methylene chloride alone. In cases where incomplete coupling occurs, the coupling procedure is repeated before removal of the α-amino protecting group, prior to the coupling of the next amino acid in the solid phase reactor. The success of the coupling reaction at each stage of the synthesis is monitored by the ninhydrin reaction as described by E. Kaiser et al, Analyt. Biochem. 34, 595 (1970).

After the desired amino acid sequence has been obtained, the peptide is removed from the resin. This can be done by hydrolysis such as by treatment of the resin bound polypeptide with a solution of dimethyl sulfide, p-cresol and thiocresol in dilute aqueous hydrofluoric acid.

As is known in the art of solid phase peptide synthesis many of the amino acids bear functionalities requiring protection during the chain preparation. The use and selection of the appropriate protecting group is within the ability of those skilled in the art and will depend upon the amino acid to be protected and the presence of other protected amino acid residues on the peptide. The selection of such a side chain protecting group is critical in that it must be one which is not removed by cleavage during cleavage of the protecting group of the α-amino moiety. For example, suitable side chain protecting groups for lysine are benzyloxycarbonyl and substituted benzyloxycarbonyl, said substituent being selected from halo (e.g., chloro, bromo, fluoro) and nitro (e.g., 2-chlorobenzyloxycarbonyl, p-nitrobenzyloxy-carbonyl, 3,4-dichlorobenzyloxycarbonyl), tosyl, t-amyloxycarbonyl, t-butyloxycarbonyl and diisopropylmethoxycarbonyl. The alcoholic hydroxyl group of threonine and serine can be protected with an acetyl, benzoyl, tert-butyl, trityl, benzyl, 2,6-dichlorobenzyl or benzyloxycarbonyl group. The preferred protecting group is benzyl.

These groups can be removed by procedures well known in the art. Typically protecting group removal is done after the peptide chain synthesis is complete but the protecting groups can be removed at any other appropriate time.

The anticoagulant dose of a peptide derivative of this invention is from 0.2 mg/kg to 250 mg/kg of patient body weight per day depending on the patient, the severity of the thrombotic condition to be treated and the peptide derivative selected. The suitable dose for a particular patient can be readily determined. Preferably from 1 to 4 daily doses would be administered typically with from 5 mg to 100 mg of active compound per dose.

Anticoagulant therapy is indicated for the treatment and prevention of a variety of thrombotic conditions, particularly coronary artery and cerebrovascular disease. Those experienced in this field are readily aware of the circumstances requiring anticoagulant therapy. The term "patient" used herein is taken to mean mammals such as primates, including humans, sheep, horses, cattle, pigs, dogs, cats, rats and mice.

Although some of the peptide derivatives may survive passage through the gut following oral administration, applicants prefer non-oral administration, for example, subcutaneous, intravenous, intramuscular or intraperitoneal; administration by depot injection; by implant preparation; or by application to the mucous membranes, such as, that of the nose, throat and bronchial tubes, for example, in an aerosol can containing a peptide derivative of this invention in a spray or dry powder form.

For parenteral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber manufactured by the Dow-Corning Corporation.

### EXAMPLES

This invention is illustrated by the following, nonlimiting examples.

### EXAMPLE 1

### Preparation of

Suc-Tyr-Glu-Pro-Ile-Pip-Glu-Glu-Ala-Cha-D-Glu-OH

The peptide was snythesized by solid-phase methods using 0.5 mmol of a 0.56 mmol/g Boc-D-Glu(Bzl)-Merrifield resin. Double symmetrical anhydride couplings were performed with 2.0 mmol N_{α}-Boc-amino acid (Peptides International) except in the case of Boc-Pip, which was single coupled. The side chain protection utilized was: Glu(Bzl), Tyr(2-BrZ). The terminal N_{α}-Boc protection was removed with 50% trifluoroacetic acid in methylene chloride. The resin was washed three times with methylene chloride, neutralized with three washings of 10% diisopropylethylamine in methylene chloride, washed three times with methylene chloride. The peptide was end-capped with succinic anhydride (1.0 g) in dimethyl formamide, washed three times with dimethyl formamide, washed three times with methylene chloride, and tested with free amine. The capping reaction was repeated two more times and the resin dried. The peptide was deprotected and cleaved from the resin with HF containing 5% anisole at 0°C, for 45 min. The HF was removed in vacuo at 0°C, the peptide was extracted from the resin with 30% aqueous acetonitrile and lyophilized.

The peptide was purified by preparative HPLC performed on a C¹⁸ Beckman (50.8 x 150 mm) column with a 33-38% acetonitrile gradient in 0.1% aqueous TFA at 79 ml/min. The major peak was collected, lyophilized, and further purified on the same column with a 34-35.5% acetonitrile gradient in 0.1% aqueous TFA at 80 ml/min. The major peak was collected and lyophilized to give 104 mg of desired product. Preparative HPLC was performed on a C¹⁸ Beckman (50.8 x 150 mm) column with a 33-38% acetonitrile gradient in 0.1% aqueous trifluoroacetic acid at 79 ml/min. The major peak was collected and lyophilized leaving 101 mg of the desired product (58% yield based on initial resin substitution). Homogeneity was determined by HPLC and TLC. HPLC Vydac 218TP54 (250 x 4.6 mm) C¹⁸ column, 2 ml/min, tₒ = 1.9 min: time of elution with a 15-40% acetonitrile in 0.1% trifluoroacetic acid linear gradient at 1ml/min. (HPLC) is 14.4 min.

Amino acid analysis: (6N HCl hydrolysis; 24 hr. at 106°C). Glx 4.02 (4); Pro 0.99 (1); Ala 0.99 (1); Ile 0.95 (1); Tyr 0.97(1); Pip 1.08 (1); ε275 = 1490. 91.5% peptide content by weight.

In the same manner, the peptides of the following examples 2-4 were prepared.

### EXAMPLE 2

Suc-Tyr-Glu-Pip-Ile-Pro-Glu-Glu-Ala-Cha-D-Glu-OH

### EXAMPLE 3

Suc-Tyr-Glu-Pro-Ile-Azd-Glu-Glu-Ala-Cha-D-Glu-OH

### EXAMPLE 4

Suc-Tyr-Glu-Azd-Ile-Pro-Glu-Glu-Ala-Cha-D-Glu-OH

| EXAMPLE NO. | Amino Acids Analysis (6N HCl Hydrolysis: 24 Hrs at 106°C) | | | | | | |
|---|---|---|---|---|---|---|---|
| | Glx | Pro | Ala | Ile | Tyr | Pip | Peptide Content |
| 2 | 3.97(4) | 1.03(1) | 0.98(1) | 0.95(1) | 0.98(1) | 1.09(1) | 84% |
| 3 | 4.04(4) | 1.03(1) | 0.98(1) | 0.97(1) | 0.98(1) | | 82% |
| 4 | 4.01(4) | 1.04(1) | 0.99(1) | 0.96(1) | 1.00(1) | | 86% |

| Physical Characteristics | | | |
|---|---|---|---|
| EXAMPLE NO. | HPLC tᵣ (min) 15-40% gradient tₒ = 1.65 min | FAB-MS (M + H)⁺ | ε275 |
| 2 | 19.05 | 1344 | 1630 |
| 3 | 15.65 | 1316 | 1610 |
| 4 | 16.25 | 1316 | 1560 |

## Claims

1. A peptide derivative of the formula
X-A₁-A₂-A₃-A₄-A₅-A₆-A₇-A₈-A₉-A₁₀-Y
wherein
X is a hydrogen, one or two alkyl groups selected from the group methyl, ethyl, propyl, isopropyl, butyl, isobutyl, and tert-butyl, or one or two acyl groups selected from the group acetyl, and succinyl;
A₁ is a bond;
A₂ is Tyr;
A₃ is Glu;
A₄ is Pro, Pip, Azd, or Tiq;
A₅ is Ile;
A₆ is Pro, Pip, Azd, or Tiq;
A₇ is Glu;
A₈ is Glu;
A₉ is the dipeptide Ala-Cha;
A₁₀ is D-Glu; and
Y is a carboxy terminal residue selected from OH, (C₁-C₆)alkoxy, and amino, with the proviso that A₄ and A₆ cannot both be a Pro.

2. A peptide derivative of claim 1 wherein X is succinyl.

3. A peptide derivative of claim 1 wherein Y is OH.

4. A peptide derivative of claim 1 which is Suc-Tyr-Glu-Pro-Ile-Pip-Glu-Glu-Ala-Cha-D-Glu-OH

5. A peptide derivative of claim 1 which is Suc-Tyr-Glu-Pip-Ile-Pro-Glu-Glu-Ala-Cha-D-Glu-OH

6. A peptide derivative of claim 1 which is Suc-Tyr-Glu-Pro-Ile-Azd-Glu-Glu-Ala-Cha-D-Glu-OH

7. A peptide derivative of claim 1 which is Suc-Tyr-Glu-Azd-Ile-Pro-Glu-Glu-Ala-Cha-D-Glu-OH

8. Use of a peptide derivative of any one of claims 1-7 for the preparation of a pharmaceutical composition for reducing blood coagulation in a patient in need thereof.

9. A method for the preparation of a peptide derivative as defined in any one of claims 1-7 by solid phase sequential or block synthesis, gene cloning or a combination thereof.

10. The process according to claim 9 whereby a solid phase sequential or block synthesis is performed, comprising binding a suitably protected amino acid of formula A₁, as defined in claim 1, to an activated resin support, subsequently binding the other alpha amino protected amino acids of the formula A₂-A₁₀, as defined in claim 1, to the terminal amino acid group of the peptide chain, which has meanwhile been exposed by removing its amino protecting group.

## Patentansprüche

1. Peptidderivat der Formel
X-A₁-A₂-A₃-A₄-A₅-A₆-A₇-A₈-A₉-A₁₀-Y
in der
X ein Wasserstoffatom, einen oder zwei Alkylreste, die aus einer Methyl-, Ethyl-, Propyl-, Isopropyl-, Butyl-, Isobutyl- und tert.-Butylgruppe ausgewählt werden, oder einen oder zwei Acylreste bedeutet, die aus einer Acetyl- und Succinylgruppe ausgewählt werden;
A₁ eine Bindung darstellt;
A₂ Tyr bedeutet;
A₃ Glu darstellt;
A₄ Pro, Pip, Azd oder Tiq bedeutet;
A₅ Ile darstellt;
A₆ Pro, Pip, Azd oder Tiq bedeutet;
A₇ Glu darstellt;
A₈ Glu bedeutet;
A₉ das Dipeptid Ala-Cha darstellt;
A₁₀ D-Glu bedeutet und
Y einen C-terminalen Rest darstellt, der aus einer OH-Gruppe, einem (C₁-C₆)-Alkoxyrest und einer Aminogruppe ausgewählt wird, mit der Maßgabe, daß A₄ und A₆ nicht beide eine Pro-Gruppe sein können.

2. Peptidderivat nach Anspruch 1, wobei X eine Succinylgruppe darstellt.

3. Peptidderivat nach Anspruch 1, wobei Y eine OH-Gruppe bedeutet.

4. Peptidderivat nach Anspruch 1, nämlich Suc-Tyr-Glu-Pro-Ile-Pip-Glu-Glu-Ala-Cha-D-Glu-OH.

5. Peptidderivat nach Anspruch 1, nämlich Suc-Tyr-Glu-Pip-Ile-Pro-Glu-Glu-Ala-Cha-D-Glu-OH.

6. Peptidderivat nach Anspruch 1, nämlich Suc-Tyr-Glu-Pro-Ile-Azd-Glu-Glu-Ala-Cha-D-Glu-OH.

7. Peptidderivat nach Anspruch 1, nämlich Suc-Tyr-Glu-Azd-Ile-Pro-Glu-Glu-Ala-Cha-D-Glu-OH.

8. Verwendung eines Peptidderivats nach einem der Ansprüche 1 - 7 zur Herstellung eines Arzneimittels zur Verminderung der Blutgerinnung bei einem Patienten, der dessen bedarf.

9. Verfahren zur Herstellung eines Peptidderivats, wie in einem der Ansprüche 1 - 7 definiert, durch Festphasensequenz- oder -blocksynthese, Genklonierung oder eine Kombination davon.

10. Verfahren nach Anspruch 9, bei dem eine Festphasensequenz- oder -blocksynthese durchgeführt wird, das die Bindung einer geeignet geschützten Aminosäure der Formel A₁, wie in Anspruch 1 definiert, an einen aktivierten Harzträger und anschließend die Bindung der anderen α-aminogeschützten Aminosäuren der Formel A₂ - A₁₀, wie in Anspruch 1 definiert, an die endständige Aminosäuregruppe der Peptidkette umfaßt, die inzwischen einer Entfernung ihrer Aminoschutzgruppen ausgesetzt worden war.

## Revendications

1. Dérivé peptidique de formule
X-A₁-A₂-A₃-A₄-A₅-A₆-A₇-A₈-A₉-A₁₀-Y
où
X est un hydrogène, un ou deux groupes alkyle choisis dans le groupe de méthyle, éthyle, propyle, isopropyle, butyle, isobutyle et tert-butyle, ou un ou deux groupes acyle choisis dans le groupe de acétyle et succinyle ;
A₁ est une liaison ;
A₂ est Tyr;
A₃ est Glu ;
A₄ est Pro, Pip, Azd ou Tiq;
A₅ est Ile ;
A₆ est Pro, Pip, Azd ou Tiq ;
A₇ est Glu ;
A₈ est Glu ;
A₉ est un dipeptide Ala-Cha;
A₁₀ est D-Glu ; et
Y est un résidu carboxy-terminal choisi parmi OH, alcoxy en C₁-C₆ et amino, à condition que A₄ et A₆ ne puissent pas être tous deux un Pro.

2. Dérivé peptidique selon la revendication 1, où X est succinyle.

3. Dérivé peptidique selon la revendication 1, où Y est OH.

4. Dérivé peptidique selon la revendication 1, qui est Suc-Tyr-Glu-Pro-Ile-Pip-Glu-Glu-Ala-Cha-D-Glu-OH.

5. Dérivé peptidique selon la revendication 1. qui est Suc-Tyr-Glu-Pip-lle-Pro-Glu-Glu-Ala-Cha-D-Glu-OH.

6. Dérivé peptidique selon la revendication 1, qui est Suc-Tyr-Glu-Pro-Ile-Azd-Glu-Glu-Ala-Cha-D-Glu-OH.

7. Dérivé peptidique selon la revendication 1, qui est Suc-Tyr-Glu-Azd-Ile-Pro-Glu-Glu-Ala-Cha-D-Glu-OH.

8. Utilisation d'un dérivé peptidique selon l'une quelconque des revendications 1 à 7 pour la préparation d'une composition pharmaceutique pour réduire la coagulation du sang chez un patient qui en a besoin.

9. Procédé pour la préparation d'un dérivé peptidique selon l'une quelconque des revendications 1 à 7, par synthèse séquentielle ou par blocs en phase solide, clonage de gènes ou une combinaison de ceux-ci.

10. Procédé selon la revendication 9, dans lequel une synthèse séquentielle ou par blocs en phase solide est réalisée, qui comprend la liaison d'un acide aminé protégé de manière appropriée de formule A₁, comme défini dans la revendication 1, avec un support de résine activée, puis la liaison des autres acides aminés α-amino-protégés de formule A₂-A₁₀, comme défini dans la revendication 1, avec le groupe acide aminé terminal de la chaîne peptidique, qui a entre temps été exposé par retrait de son groupe amino-protecteur.
